# EUROPEAN PATENT SPECIFICATION

(11) **EP 3 177 263 B1**
(45) Date of publication and mention of the grant of the patent: **28.10.2020**
(21) Application number: 15749789.2
(22) Date of filing: 07.08.2015
(51) Int. Cl.: A61K 8/25, A61K 8/36, A61Q 17/04

(54) **SOAP BASED SUNSCREEN COMPOSITION**
AUF SEIFE BASIERENDES SONNENSCHUTZMITTEL
COMPOSITION D'ÉCRAN SOLAIRE À BASE DE SAVON

(30) Priority: 08.08.2014 IN 2273DE2014
(43) Date of publication of application: 14.06.2017
(73) Proprietor: L'Oréal, 75008 Paris (FR)
(72) Inventor: GHOSH, Nilmoni, West Bengal 741101 (IN); BURSHE, Mahesh, Maharashtra 400606 (IN)
(74) Representative: Lavoix
(86) International application number: PCT/EP2015/068312
(87) International publication number: WO 2016/020541

(56) References cited:
- WO-A1-2013/117449
- FR-A1- 2 986 423
- US-A1- 2013 287 828
- US-A1- 2013 337 026

## Description

The present invention relates to a composition able to provide UV protection, for example to the skin. The present invention particularly relates to a cosmetic composition.

It is known to use compositions able to provide oil control performance, notably when applied to the skin.

However, sunscreen compositions (i.e. compositions having UV protection) generally include high level of lipophilic UV absorbing agents, which add up to the oil phase of those compositions. These lipophilic UV absorbing agents are applied to form a film able to provide a long-lasting protection from UV radiations.

FR 2 986 423 discloses compositions comprising at least one oil phase comprising at least one polar oil and at least one lipophilic UV filter, and hydrophobic silica aerogel particles. In particular, these compositions comprise 1.5% by weight of stearic acid.

However, a problem of these sunscreen compositions is that they usually leave an oily, sticky, shiny and/or unpleasant residue. Accordingly, sunscreen compositions are generally poor in terms of oil control, skin mattifying effect and skin shine reduction.

There is thus a need for improving the oil control of the sunscreen compositions.

There is also a need for sunscreen cosmetic compositions which afford a long-lasting mattifying effect to the skin.

There is also a need for sunscreen cosmetic compositions able to reduce the shine of the skin.

The aim of the present invention is to provide a sunscreen composition, comprising high level of lipophilic UV absorbing agent, which does not have the drawbacks of the sunscreen compositions of the prior art.

Notably, the aim of the present invention is to provide a sunscreen composition having a long lasting oil and sweat control performance.

The aim of the present invention is also to provide a sunscreen cosmetic composition able to mattify the skin and/or reduce the shine of the skin.

The Applicant has discovered that these needs could be met by combining at least one fatty acid soap and some hydrophobic silica aerogel particles, in a sunscreen composition further comprising at least one lipophilic UV absorption agent and an aqueous phase.

When it is applied to the skin, the composition thus obtained makes it possible to improve skin mattness and/or to reduce the skin shine in a lasting manner. The skin, while being protected from the UV radiations damages, is thus rendered matt and/or less shiny in a long-lasting manner. The composition of the invention also provides a long lasting oil and sweat control performance by reducing the production of sebum of the skin, particularly in tropical climate.

The present invention relates to a composition comprising:
a) one or more fatty acids selected from the group consisting of straight or branched saturated fatty acids in C₈-C₃₀,
b) at least one basic agent,
c) at least one lipophilic UV absorption agent,
d) at least hydrophobic silica aerogel particles, and
e) at least one aqueous phase,
wherein said fatty acid(s) is(are) preset in the composition in an amount comprised from 4% to 25% by weight relative to the total weight of the composition.

The composition of the invention is able to provide a long-lasting UV protection without leaving any oily, sticky, shiny and/or unpleasant residue, particularly when it is applied to the skin. Advantageously, the composition of the invention is able to provide a long-lasting UVA, UVB or UVA/UVB protection without leaving any oily, sticky, shiny and/or unpleasant residue, particularly when it is applied to the skin.

The composition of the invention may be in any cosmetic form conventionally used for a topical application and especially in the form of dispersions of gel or lotion type, emulsions of liquid or semi-liquid consistency of the milk type, obtained by dispersing an oil phase in an aqueous phase (O/W) or vice versa (W/O), or suspensions or emulsions of soft, semi-solid or solid consistency of the cream or gel type, or alternatively multiple emulsions (W/O/W or O/W/O), microemulsions, vesicular dispersions of ionic and/or non-ionic type, or wax/aqueous phase dispersions. These compositions are prepared according to the usual methods.

In addition, the composition of the invention can be more or less fluid and can have the appearance of a white or coloured cream, of an ointment, of a milk, of a lotion, of a serum, of a paste or of a foam. It may optionally be applied to the skin in the form of an aerosol. It can also be in solid form, for example in the form of a stick.

The composition of the invention is preferably in the form of a gel or a cream.

### Fatty acids

The composition of the invention comprises a) one or more fatty acids selected from the group consisting of the straight or branched saturated fatty acids in C₈-C₃₀.

The composition of the invention may comprise a mixture of fatty acids.

According to one embodiment, the fatty acid is in C₁₂-C₂₂, preferably in C₁₂-C₂₀, advantageously in C₁₄-C₁₈.

According to one embodiment, the fatty acid is selected from palmitic acid (C₁₆), stearic acid (C₁₈), myristic acid (C₁₄), lauric acid (C₁₂), and mixtures thereof.

According to a preferred embodiment, the fatty acid is stearic acid.

According to another preferred embodiment, the fatty acid is a mixture of stearic acid, palmitic acid and myristic acid, said mixture comprising from 50% to 60% of stearic acid, from 40% to 50% of palmitic acid, and from 1% to 5% of myristic acid, by weight relative to the total weight of said mixture.

According to one embodiment, the composition comprises at least one fatty acid in C₁₆-C₃₀, preferably in C₁₆-C₂₂, notably selected from palmitic acid, stearic acid, and mixture thereof. In this embodiment, the fatty acids in C₈-C₁₄ that may be present in addition to the fatty acids in C₁₆-C₃₀ are preferably present in an amount less than or equal to 2% by weight relative to the total weight of the composition. Said amount may be comprised from 0% to 2% by weight, preferably from 0.0001% to 2%, more preferably from 0.0001% to 1.5% by weight relative to the total weight of the composition.

According to the invention, the fatty acid is present in the composition in an amount comprised from 4% to 25%, more preferably from 5% to 20%, advantageously from 5% to 15%, by weight relative to the total weight of the composition.

In the composition of the invention, a portion of the fatty acids is saponified (also called neutralized, or in the form of a soap) by the basic agent b).

The amount of neutralized fatty acid is preferably comprised from 3% to 50%, more preferably from 5% to 30%, advantageously from 8% to 20% by weight relative to the total weight of fatty acids present in the composition. The other portion of the fatty acid remains in the free form.

Preferably, the fatty acid or the mixture of fatty acids is saponified *in situ* by adding a desired amount of basic agent to the fatty acid or the mixture of fatty acids.

### Basic agent

The composition of the invention comprises b) at least one basic agent.

According to one embodiment, the basic agent is selected from the group consisting of alkali metal hydroxides such as sodium hydroxide and potassium hydroxide; alkaline earth metal hydroxides such as magnesium hydroxide; ammonium hydroxide; organic bases, such as amines and alkanolamines; and mixtures thereof.

Examples of amines or alkanolamines suitable for use according to said variant include, but are not limited to, monoethanolamine, diethanolamine, triethanolamine, monoisopropanolamine, monoisopropylamine, isopropylamine, N-propylamine, diethyleneamine, thriethylamine, N-butylamine, isobutylamine, cyclohexylamine, N-methylglucamine, lysine, and arginine, and mixtures thereof.

Preferably, the basic agent is triethanolamine.

According to a preferred embodiment, the fatty acid is stearic acid and the basic agent is triethanolamine.

The basic agent is generally present in the composition in an amount comprised from 0.1% to 10%, preferably from 0.1% to 8%, advantageously from 0.1% to 5%, specifically from 0.1% to 3%, by weight relative to the total weight of the composition.

The basic agent is preferably present in an amount sufficient so that the pH of the composition is comprised from 6 to 9, preferably from 7 to 8.

The basic agent is preferably present in an amount sufficient so that a portion of the fatty acid remains in the free form.

The basic agent is preferably present in the aqueous phase of the composition.

### UV absorption agent

The composition of the invention comprises c) at least one lipophilic UV absorption agent.

The composition of the invention preferably comprises a mixture of lipophilic UV absorption agents. Said mixture of UV absorption agents is generally formed of organic UV absorption agents or a mixture of organic and mineral UV absorption agents.

According to one embodiment, the composition of the invention comprises a mixture of at least one UVA absorption agent and at least one UVB absorption agent.

The composition of the invention generally comprises a mixture of UV absorption agents, said mixture being preferably present in the composition in an amount comprised from 1% to 25%, advantageously from 5% to 15%, more specifically from 7.5% to 12.5%, by weight relative to the total weight of the composition.

### Organic UV absorption agents

The organic UV absorption agents may be lipophilic, hydrophilic or insoluble.

Within the framework of the present invention, the term "lipophilic UV absorption agent" means an organic compound able to block UV radiations, said compound being completely dissolved in the liquid oily phase of a composition or being solubilized in a colloidal form in the liquid oily phase of a composition.

Within the framework of the present invention, the term "hydrophilic UV absorption agent" means an organic compound able to block UV radiations, said compound being completely dissolved in the liquid aqueous phase of a composition or being solubilized in a colloidal form in the liquid aqueous phase of a composition.

Within the framework of the present invention, the term "insoluble UV absorption agent" means an organic compound able to block UV radiations, said compound having a solubility in water less than 0.5% in weight and a solubility less than 0.5% in weight in most of organic solvents, such as paraffin oils, fatty alcohol benzoates and fatty acids triglycerids. Said solubility is assessed at 70°C and is defined as the amount of product in solution in the solvent in equilibrium with an excess of solid in suspension after the temperature came back to room temperature. Said solubility can be easily assessed in laboratory.

The lipophilic organic UV absoprtion agents are chosen especially from cinnamic derivatives; anthranilates; salicylic derivatives; dibenzoylmethane derivatives, camphor derivatives; benzophenone derivatives; β,β-diphenylacrylate derivatives; triazine derivatives; benzotriazole derivatives; benzalmalonate derivatives, especially those mentioned in patent US 5 624 663; imidazolines; p-aminobenzoic acid (PABA) derivatives; benzoxazole derivatives as described in patent applications EP 0 832 642, EP 1 027 883, EP 1 300 137 and DE 101 62 844; screening polymers and screening silicones such as those described especially in patent application WO 93/04665; cc-alkylstyrene-based dimers such as those described in patent application DE 198 55 649; 4,4-diarylbutadienes such as those described in patent applications EP 0 967 200, DE 197 46 654, DE 197 55 649, EP-A-1 008 586, EP 1 133 980 and EP 1 133 981 ; merocyanin derivatives such as those described in patent applications WO 04/006 878, WO 05/058 269, WO 06/032 741 , FR 2 957 249 and FR 2 957 250; and mixtures thereof.

As examples of lipophilic organic UV absorption agents, mention may be made of those denoted hereinbelow under their INCI name:
Dibenzoylmethane derivatives:
   Butyl Methoxy Dibenzoylmethane or avobenzone, provided for sale under the trade name Parsol 1789 by DSM Nutritional Products,
para-Aminobenzoic acid derivatives:
   PABA,
   Ethyl PABA,
   Ethyl Dihydroxypropyl PABA,
   Ethylhexyl Dimethyl PABA sold in particular under the name Escalol 507 by ISP,
Salicylic derivatives:
   Homosalate sold under the name Eusolex HMS by Rona/EM Industries,
   Ethylhexyl salicylate sold under the name Neo Heliopan OS by Symrise,
Cinnamic derivatives:
   Ethylhexyl methoxycinnamate sold especially under the trade name Parsol MCX by DSM Nutritional Products,
   Isopropyl methoxycinnamate,
   Isoamyl p-methoxycinnamate sold under the trade name Neo Heliopan E 1000 by Symrise,
   Cinoxate,
   Diisopropyl Methylcinnamate,
β,β-Diphenylacrylate derivatives:
   Octocrylene sold especially under the trade name Uvinul N539 by BASF,
   Etocrylene sold in particular under the trade name Uvinul N35 by BASF,
Benzophenone derivatives:
   Benzophenone-1 sold under the trade name Uvinul 400 by BASF,
   Benzophenone-2 sold under the trade name Uvinul D50 by BASF,
   Benzophenone-3 or oxybenzone sold under the trade name Uvinul M40 by BASF,
   Benzophenone-6 sold under the trade name Helisorb 1 1 by Norquay,
   Benzophenone-8 sold under the trade name Spectra-Sorb UV-24 by American Cyanamid,
   Benzophenone-12,
   n-hexyl 2-(4-diethylamino-2-hydroxybenzoyl)benzoate sold under the trade name Uvinul A+ or in the form of a mixture with octyl methoxycinnamate under the trade name Uvinul A+B by BASF, 1,1'-(1,4-piperazinediyl)bis[1-[2-[4-(diethylamino)-2-hydroxybenzoyl]phenyl]methanone (CAS 919803-06-8) in micronized or non- micron ized form,
Benzvlidene camphor derivatives:
   3-Benzylidene Camphor manufactured under the name Mexoryl SD by Chimex,
   4-Methylbenzylidene Camphor sold under the name Eusolex 6300 by Merck,
   Polyacrylamidomethylbenzylidenecamphor manufactured under the name Mexoryl SW by Chimex.
Phenylbenzotriazole derivatives:
   Drometrizole trisiloxane sold under the name Silatrizole by Rhodia Chimie,
Triazine derivatives:
   bis-Ethylhexyloxyphenol methoxyphenyl triazine sold under the trade name Tinosorb S by BASF,
   Ethylhexyl triazone sold in particular under the trade name Uvinul T150 by BASF, Diethylhexyl Butamido Triazone sold under the trade name Uvasorb HEB by Sigma 3V,
   2,4,6-Tris(dineopentyl 4'-aminobenzalmalonate)-s-triazine,
   2,4,6-Tris(diisobutyl 4'-aminobenzalmalonate)-s-triazine,
   2,4-Bis(dineopentyl 4'-aminobenzalmalonate)-6-(n-butyl 4'-aminobenzoate)-s-triazine,
Anthranilic derivatives:
   Menthyl anthranilate sold under the trade name Neo Heliopan MA by Symrise,
Imidazoline derivatives:
   Ethylhexyl Dimethoxybenzylidene Dioxoimidazoline Propionate,
Benzalmalonate derivatives:
   Dineopentyl 4'-methoxybenzalmalonate,
   Polyorganosiloxane containing benzalmalonate functions, for instance Polysilicone-15, sold under the trade name Parsol SLX by DSM,
4,4-Diarylbutadiene derivatives:
   1,1-Dicarboxy(2,2'-dimethylpropyl)-4,4-diphenylbutadiene,
Benzoxazole derivatives:
   2,4-Bis[4-[5-(1,1-dimethylpropyl)benzoxazol-2-yl]phenylimino]-6-[(2-ethylhexyl)imino]-1,3,5-triazine, sold under the name of Uvasorb K2A by Sigma 3V,
Lipophilic merocvanin derivatives:
   - Octyl 5-N,N-diethylamino-2-phenylsulfonyl-2,4-pentadienoate and mixtures thereof.

The preferred lipophilic organic UV absoprtion agents are chosen from:
Butyl Methoxy Dibenzoylmethane, Ethylhexyl Methoxycinnamate,
Ethylhexyl salicylate,
Homosalate,
Butylmethoxydibenzoylmethane,
Octocrylene,
Benzophenone-3,
n-Hexyl 2-(4-diethylamino-2-hydroxybenzoyl)benzoate,
4-Methylbenzylidene Camphor,
Bis-Ethylhexyloxyphenol Methoxyphenyl Triazine,
Ethylhexyl Triazone,
Diethylhexyl Butamido Triazone,
2,4,6-Tris(dineopentyl 4'-aminobenzalmalonate)-s-triazine,
2,4,6-Tris(diisobutyl 4'-aminobenzalmalonate)-s-triazine,
2,4-Bis(di-neopentyl 4'-aminobenzalmalonate)-6-(n-butyl 4'-aminobenzoate)-s-triazine,
Drometrizole Trisiloxane,
Polysilicone-15,
1,1 -Dicarboxy(2,2'-dimethylpropyl)-4,4-diphenylbutadiene,
2,4-Bis[4-[5-(1,1-dimethylpropyl)benzoxazol-2-yl]phenylimino]-6-[(2-ethylhexyl)imino]-1,3,5-triazine,
and mixtures thereof.

The preferred lipophilic organic screening agents are more particularly chosen from:
Butyl Methoxy Dibenzoylmethane (avobenzone),
Octocrylene,
Ethylhexyl Salicylate,
n-Hexyl 2-(4-diethylamino-2-hydroxybenzoyl)benzoate,
Bis-Ethylhexyloxyphenol Methoxyphenyl Triazine,
Ethylhexyl Triazone,
Diethylhexyl Butamido Triazone,
Drometrizole Trisiloxane, and mixtures thereof.

The lipophilic organic UV-screening agent(s) are preferably present in the composition according to the invention in an amount comprised from 0.1 % to 25%, more preferably from 1% to 20%, advantageously from 5% to 15%, by weight relative to the total weight of the composition.

According to one embodiment, the lipophilic UV absorption agent present in the composition is selected from the group consisting of dibenzoylmethane derivatives, β,β-diphenylacrylate derivatives, phenyl benzotriazole derivatives, and mixtures thereof.

When the lipophilic UV absorption agent is a dibenzoylmethane derivative, such as avobenzone, it is preferably present in the composition in an amount comprised from 0.4% to 10%, more preferably from 3% to 4%, by weight relative to the total weight of the composition.

When the lipophilic UV absorption agent is a β,β-diphenylacrylate derivative, such as octocrylene, it is preferably present in the composition in an amount comprised from 2% to 10%, more preferably from 4.5% to 10%, by weight relative to the total weight of the composition.

When the lipophilic UV absorption agent is a phenyl benzotriazole derivative, such as drometrizole trisiloxane, it is preferably present in the composition in an amount comprised from 0.1% to 10%, more preferably from 1% to 5%, by weight relative to the total weight of the composition.

Preferably, the lipophilic UV absorption agent is avobenzone, also called butyl methoxydibenzoylmethane, notably commercialized by DSM Nutritial Products under the name PARSOL 1789®.

Preferably, the lipophilic UV absorption agent is octocrylene, also called 2-ethylhexyl α-cyano-β,β'-diphenylacrylate, notably commercialized by BASF under the name UVINUL N 539®.

Preferably, the lipophilic UV absorption agent is drometrizole trisiloxane, notably commercialized by CHIMEX under the name MEXORYL XL®.

According to one embodiment, the composition of the invention further comprises at least one hydrophilic UV absorption agent.

Said hydrophilic UV absorption agent is preferably selected from phenyl benzimidazole derivatives, such as phenyl benzimidazole sulphonic acid, notably commercialized by MERCK under the name EUSOLEX 232®.

When the composition of the invention comprises one hydrophilic UV absorption agent, such as phenyl benzimidazole sulphonic acid, it is usually present in an amount comprised from 0.1% to 10%, more preferably from 1% to 5%, by weight relative to the total weight of the composition.

According to one embodiment, the composition of the invention comprises a mixture of octocrylene, avobenzone, drometrizole trisiloxane and phenyl benzimidazole sulphonic acid.

### Mineral UV absorption agents

The mineral UV absorption agents are also called pigments.

Preferably, the mineral UV absorption agents are in the form of particles of metal oxide having a mean size less or equal to 0.5 µm, preferably comprised from 0.005 µm and 0.5 µm, more preferably comprised from 0.01 µm and 0.2 µm, even more preferably from 0.01 µm and 0.1 µm, advantageously from 0.015 µm and 0,05 µm.

Non-limiting examples of mineral UV absorption agents include kaolin, talc, petrolatum and metal oxides (such as titanium dioxide, zinc oxide, iron oxide, zirconium oxide, or cerium oxide).

### Hydrophobic silica aerogel particles

The composition of the invention comprises hydrophobic silica aerogel particles.

Silica aerogels are porous materials obtained by replacing (especially by drying) the liquid component of a silica gel with air.

They are generally synthesized via a sol-gel process in a liquid medium and then dried, usually by extraction with a supercritical fluid, the one most commonly used being supercritical CO₂. This type of drying makes it possible to avoid shrinkage of the pores and of the material. The sol-gel process and the various drying operations are described in detail in Brinker C.J. and Scherer G.W., Sol-Gel Science, New York: Academic Press, 1990.

The term "hydrophobic aerogel particle" means any particle of the aerogel type having a water absorption capacity at the wet point of less than 0.1 ml/g, i.e. less than 10 g of water per 100 g of particle.

The wet point corresponds to the amount of water that needs to be added to 1 g of particle in order to obtain a homogeneous paste. This method is derived directly from the method for determining the oil uptake of a powder as described in standard NF T 30-022. The measurements are taken in the same manner by means of the wet point and the flow point, which have, respectively, the following definitions:
wet point: weight expressed in grams per 100 g of product corresponding to the production of a homogeneous paste during the addition of a solvent to a powder.

The wet point is measured according to the following protocol:
Equipment used:
Glass plate (25 x 25 mm)
Spatula (wooden shaft and metal part, 15 x 2.7 mm)
Silk-bristled brush
Balance

The glass plate is placed on the balance and 1 g of aerogel is weighed out. The beaker containing the solvent and the liquid sampling pipette is placed on the balance. The solvent is gradually added to the powder, the whole being regularly blended (every 3 to 4 drops) with the spatula. The mass of solvent required to reach the wet point is noted. The average of three tests will be determined.

The hydrophobic silica aerogel particles used in the present invention have a specific surface area per unit of mass (S_{M}) ranging from 200 m²/g to 1500 m²/g, preferably from 600 m²/g to 1200 m²/g and better still from 600 m²/g to 800 m²/g, and preferably a size, expressed as the volume-mean diameter (D[0.5]), of less than 1500 µm and preferably ranging from 1 µm to 30 µm, more preferably from 5 µm to 25 µm, better still from 5 µm to 20 µm and even better still from 5 µm to 15 µm.

The specific surface area per unit of mass can be determined by the nitrogen absorption method, known as the BET (Brunauer-Emmett-Teller) method, described in The Journal of the American Chemical Society, Vol. 60, page 309, February 1938 and corresponding to the international standard ISO 5794/1 (appendix D). The BET specific surface area corresponds to the total specific surface area of the particles under consideration.

The sizes of the aerogel silica particles according to the invention can be measured by static light scattering using a commercial particle size analyser such as the MasterSizer 2000 machine from Malvern. The data are processed on the basis of the Mie scattering theory. This theory, which is exact for isotropic particles, makes it possible to determine, in the case of non-spherical particles, an "effective" particle diameter. This theory is especially described in the publication by Van de Hulst, H.C., "Light Scattering by Small Particles", Chapters 9 and 10, Wiley, New York, 1957.

According to an advantageous embodiment, the hydrophobic silica aerogel particles used in the present invention have a specific surface area per unit of mass (S_{M}) ranging from 600 m²/g to 800 m²/g and a size, expressed as the volume-mean diameter (D[0.5]), ranging from 5 µm to 20 µm and better still from 5 µm to 15 µm.

The hydrophobic silica aerogel particles used in the present invention may advantageously have a tapped density ρ ranging from 0.02 g/cm³ to 0.10 g/cm³ and preferably from 0.02 g/cm³ to 0.08 g/cm³.

In the context of the present invention, this density may be assessed according to the following protocol, known as the tapped density protocol:
40 g of powder are poured into a measuring cylinder; the measuring cylinder is then placed on the Stav 2003 machine from Stampf Volumeter; the measuring cylinder is subsequently subjected to a series of 2500 tapping actions (this operation is repeated until the difference in volume between 2 consecutive tests is less than 2%); and then the final volume Vf of tapped powder is measured directly on the measuring cylinder. The tapped density is determined by the ratio m/Vf, in this instance 40/Vf (Vf being expressed in cm³ and m in g).

According to one embodiment, the hydrophobic silica aerogel particles used in the present invention have a specific surface area per unit of volume Sv ranging from 5 m²/cm³ to 60 m²/cm³, preferably from 10 m²/cm³ to 50 m²/cm³ and better still from 15 m²/cm³ to 40 m²/cm³.

The specific surface area per unit of volume is given by the relationship: S_{V} = S_{M}. ρ where ρ is the tapped density expressed in g/cm³ and S_{M} is the specific surface area per unit of mass expressed in m²/g, as defined above.

Preferably, the hydrophobic silica aerogel particles according to the invention have an oil-absorbing capacity, measured at the wet point, ranging from 5 ml/g to 18 ml/g, preferably from 6 ml/g to 15 ml/g and better still from 8 ml/g to 12 ml/g.

The absorption capacity measured at the wet point, denoted Wp, corresponds to the amount of oil that needs to be added to 100 g of particles in order to obtain a homogeneous paste.

It is measured according to the "wet point" method or method for determining the oil uptake of a powder as described in standard NF T 30-022. It corresponds to the amount of oil adsorbed onto the available surface of the powder and/or absorbed by the powder by measurement of the wet point, described below:
An amount m = 2 g of powder is placed on a glass plate and the oil (isononyl isononanoate) is then added dropwise. After addition of 4 to 5 drops of oil to the powder, mixing is performed using a spatula, and addition of oil is continued until conglomerates of oil and powder have formed. From this point, the oil is added one drop at a time and the mixture is then triturated with the spatula. The addition of oil is stopped when a firm and smooth paste is obtained. This paste must be able to be spread over the glass plate without cracks or the formation of lumps. The volume Vs (expressed in ml) of oil used is then noted.

The oil uptake corresponds to the ratio Vs/m.

The hydrophobic silica aerogels particles used according to the present invention are preferably silylated silica aerogels particles (INCI name: silica silylate).

The term "silylated silica" means any silica whose surface is treated with silylating agents, for example halogenated silanes such as alkylchlorosilanes, siloxanes, in particular dimethylsiloxanes such as hexamethyldisiloxane, or silazanes, so as to functionalize the OH groups with silyl groups Si-Rn, for example trimethylsilyl groups.

As regards the preparation of hydrophobic silica aerogel particles that have been surface-modified by silylation, reference may be made to document US 7470725.

According to a preferred embodiment, the hydrophobic silica aerogels particles used according to the present invention are made of the hydrophobic silica aerogel particles that have been surface-modified with trimethylsilyl groups.

As hydrophobic silica aerogels that may be used in the invention, examples that may be mentioned include the aerogel sold under the name VM-2260 (INCI name: Silica silylate) by Dow Corning, the particles of which have a mean size of about 1000 µm and a specific surface area per unit of mass ranging from 600 m²/g to 800 m²/g.

Mention may also be made of the aerogels sold by Cabot under the references Aerogel TLD 201, Aerogel OGD 201 and Aerogel TLD 203, Enova® Aerogel MT 1100 and Enova Aerogel MT 1200.

Use will more particularly be made of the aerogel sold under the name VM-2270 (INCI name: Silica silylate) by Dow Corning, the particles of which have a mean size ranging from 5-15 µm and a specific surface area per unit of mass ranging from 600 m²/g to 800 m²/g.

Use will also be made of the aerogel sold under the name Enova® Aerogel MT 1100 (INCI name: Silica silylate) by Cabot, the particles of which have a mean size ranging from 2-25 µm and a specific surface area per unit of mass ranging from 600 m²/g to 800 m²/g.

The hydrophobic silica aerogel particles represent from 0.01% to 30%, preferably from 0.01% to 20%, better still from 0.05% to 10%, more preferably from 0.1% to 5% and for example from 0.2% to 1% by weight relative to the total weight of the composition.

### Additional ingredients

The composition of the invention may comprise additional ingredients, which may bring additional properties to the composition, such as lightening properties, instant optical benefits, fairness and/or glow.

According to one embodiment, the composition of the invention comprises at least one skin lightening agent.

When the composition of the invention comprises a skin lightening agent, it is generally present in an amount comprised from 0.01% to 15%, preferably from 0.1% to 10%, by weight relative to the total weight of the composition.

The skin lightening agent is preferably chosen from the group consisting of vitamin B3; derivatives of vitamin B3; and other well-known skin lightening agents such as adapalene, aloe extract, ammonium lactate, anethole derivatives, apple extract, arbutin, azelaic acid, kojic acid, bamboo extract, bearberry extract, bletilla tuber, bupleurum falcatum extract, burnet extract, butyl hydroxy anisole, butyl hydroxy toluene, citrate esters, Chuanxiong, Dang-Gui, deoxyarbutin, 1,3-diphenylpropane derivatives, 2,5-dihydroxybenzoic acid and its derivatives, 2-(4-acetoxyphenyl)-1,3-dithane, 2-(4-hydroxyphenyl)-1,3-dithane, ellagic acid, escinol, estragole derivatives, Fadeout (available from Pentapharm), Fangfeng, fennel extract, ganoderma extract, gaoben, Gatuline Whitening (available from Gattlefosse), genistic acid and its derivatives, glabridin and its derivatives, gluco pyranosyl-1-ascorbate, gluconic acid, glycolic acid, green tea extract, 4-hydroxy-5-methyl-3[2H]-furanone, hydroquinone, 4-hydroxyanisole and its derivatives, 4-hydroxybenzoic acid derivatives, hydroxycaprylic acid, inositol ascorbate, kojic acid, lactic acid, lemon extract, linoleic acid, magnesium ascorbyl phosphate, Melawhite (available from Pentapharm), morus alba extract, mulberry root extract, 5-octanoyl salicylic acid, parsley extract, phellinus linteus extract, pyrogallol derivatives, 2,4-resorcinol derivatives, 3,5-resorcinol derivatives, rose fruit extract, salicylic acid, Song-Yi extract, 3,4,5-trihydroxybenzyl derivatives, tranexamic acid, vitamin B6, vitamin B12, vitamin C, vitamin A, dicarboxylic acids, resorcinol derivatives, extracts from plants viz. rubia and symplocos, hydroxycarboxylic acids like lactic acid and their salts e.g. sodium lactate; and mixtures thereof.

According to a preferred embodiment, the skin lightening agent is vitamin B3, a vitamin B3 derivative, or a mixture thereof.

Examples of vitamin B3 derivatives suitable for use according to said embodiment include, but are not limited to, niacin, nicotinic acid and niacinamide.

According to a preferred embodiment, the composition of the invention comprises niacinamide. When the composition of the invention comprises niacinamide, it is generally present in an amount comprised from 0.01% to 15%, preferably from 0.1% to 10%, more preferably from 1% to 5%, by weight relative to the total weight of the composition.

According to one embodiment, the composition of the invention further comprises at least one filler other than the hydrophobic silica aerogel particles.

When the composition of the invention comprises said filler, it is generally present in an amount comprised from 0.01% to 10%, preferably from 0.05% to 8%, particularly from 0.1% to 5%, by weight relative to the total weight of the composition.

The filler is preferably chosen from the group consisting of silica other than hydrophobic silica aerogel particles, starch, talc, perlite, and mixtures thereof.

According to one embodiment, the composition of the invention further comprises silica particles other than hydrophobic silica aerogel particles.

These silica particles are different from the hydrophobic silica aerogel particles.

Silicas that can be used may be natural and untreated. Mention may thus be made of the silicas provided under the names Sillitin N85, Sillitin N87, Sillitin N82, Sillitin V85 and Sillitin V88 by Hoffmann Mineral.

They may be fumed silicas.

The fumed silicas can be obtained by high-temperature hydrolysis of a volatile silicon compound in an oxyhydrogen flame, producing a finely divided silica. This process makes it possible especially to obtain hydrophilic silicas which contain a large number of silanol groups at their surface. It is possible to chemically modify the surface of the said silica via a chemical reaction which brings about a reduction in the number of silanol groups. It is possible especially to substitute silanol groups with hydrophobic groups; a hydrophobic silica is then obtained.

The hydrophobic groups can be:
(a) trimethylsiloxyl groups, which are obtained especially by treating fumed silica in the presence of hexamethyldisilazane. Silicas thus treated are known as "Silica silylate" according to the CTFA (6th Edition, 1995);
(b) dimethylsilyloxyl or polydimethylsiloxane groups, which are obtained especially by treating fumed silica in the presence of polydimethylsiloxane or dimethyldichlorosilane. Silicas thus treated are known as "Silica dimethyl silylate" according to the CTFA (6th Edition, 1995).

Silica powders that may more particularly be mentioned include:
- the porous silica microspheres sold under the name Silica Beads SB-700 by Miyoshi; Sunsphere H51, Sunsphere H33 by Asahi Glass;
- the polydimethylsiloxane-coated amorphous silica microspheres sold under the name SA Sunsphere H33 and SA Sunsphere H53 by Asahi Glass;
- the precipitated silica microspheres, for example coated with inorganic wax such as polyethylene, and sold especially under the name Acematt OK 412 by Evonik Degussa.

Silica powder is in the form of particles having a mean particle size preferably comprised from 3 µm to 20 µm, more preferably from 4 µm to 6 µm. The silica preferably has an oil absorption capacity of 150 ml/100 g.

When the composition of the invention comprises silica particles other than the hydrophobic silica aerogel particles, they are generally present in an amount comprised from 0.1% to 10%, preferably from 0.2% to 5%, by weight relative to the total weight of the composition.

According to one embodiment, the composition of the invention further comprises starch.

The starches that may be used in the present invention are, for example, corn starch, potato starch, tapioca starch, rice starch, wheat starch and cassava starch.

The starches may be modified or unmodified.

A modified starch is a starch that has been modified via processes known to those skilled in the art, for instance esterification, etherification, oxidation, acid hydrolysis, crosslinking or enzymatic conversion.

Non-limiting examples of modified starches include aluminium starch octenylsuccinate, sodium starch octenylsuccinate, calcium starch octenylsuccinate, distarch phosphate, hydroxyethyl starch phosphate, hydroxypropyl starch phosphate, sodium carboxymethyl starch and sodium starch glycolate.

In one particular embodiment, the starch is a starch octenylsuccinate, in particular of aluminium, the starch being from corn, wheat or rice. Mention may be made especially of the product sold by Akzo Nobel under the name Dry Flo Plus.

The starches are especially chosen from powders of unmodified starch such as corn starch.

Starch is preferably in the form of particles having a mean particle size comprised from 1 µm to 10 µm.

When the composition of the invention comprises a starch, it is generally present in an amount comprised from 0.1% to 10%, preferably from 0.2% to 5%, by weight relative to the total weight of the composition.

According to one embodiment, the composition of the invention further comprises talc.

Talcs are hydrated magnesium silicates usually further comprising aluminium silicate. The crystalline structure of talc consists in layers of brucite and layers of silica. The talc may be selected from those under the name TALC SG-2000® commercialized by Nippon Talc, LUZENAC PHARMA M® commercialized by LUZENAC, J-68BC commercialized by US Corporation and MICRO ACE-P-3® commercialized by Nippon Talc.

Talc is preferably in the form of particles having a mean particle size comprised from 1 µm to 10 µm.

When the composition of the invention comprises talc, it is generally present in an amount comprised from 0.1% to 10%, preferably from 0.2% to 5%, by weight relative to the total weight of the composition.

According to one embodiment, the composition of the invention further comprises perlite.

Perlite is a natural glass of volcanic origin, shiny light-grey or black in color, which results from the rapid cooling of lava and which is provided in the shape of small particles resembling pearls.

The perlite particles used according to the invention are especially commercially available from World Minerals Europe under the trade name Perlite P1430, Perlite P2550 or Perlite P2040. These particles are sold as mattifying agents for paints. They are presented in the form of a white powder having a crystalline silica content below 0.1% by weight.

Preferably, the perlite particles according to the invention have a particle size distribution such that at least 50% of the particles have a size of less than 25 µm, preferably of less than 20 µm. In addition, they preferably have a particle size distribution such that 90% by weight of the particles have a size of less than 55 µm and preferably of less than 40 µm. Furthermore, it is preferable for 90% by weight of the particles to have a size of greater than 5 µm.

When the composition of the invention comprises perlite, it is generally present in an amount comprised from 0.1% to 10%, preferably from 0.2% to 5%, by weight relative to the total weight of the composition.

The composition of the invention may further comprise other additional ingredients, commonly used in the skin care industry. Examples include: antioxidants, binders, biological additives, buffering agents, colorants, thickeners, polymers, astringents, fragrance, humectants, opacifying agents, conditioners, exfoliating agents, pH adjusters, preservatives, natural extracts, essential oils, skin sensates, skin soothing agents and skin healing agents.

The composition of the invention may also comprise instant optical benefit agents, such as cosmetic grades titanium dioxides, pigments, colorants, pearls, mica, boron nitride, or a mixture thereof.

Advantageously, to reinforce the mattifying effects of the composition according to the invention, it also comprises at least one active agent for caring for oily skin. This active agent is preferably chosen from desquamating agents, antimicrobial agents, antiinflammatory agents, sebum regulators and antioxidants.

It may also contain cosmetic active agents other than those for caring for oily skin, for instance moisturizers and vitamins.

Naturally, a person skilled in the art will take care to select this or these optional additional compound(s), and/or the amount thereof, such that the mattifying and shine reduction advantageous properties of the composition of the invention are not, or are not substantially, adversely affected by the envisaged addition.

The composition of the invention comprises an oil phase, which at least includes the lipophilic UV absorption agent and eventually the fatty acid in the free form when it is present.

Said oil phase usually comprises at least one oil other than the C₁₂-C₂₀ fatty acid of the soap.

For example, as oils which can be used in the composition of the invention, mention may be made of:
- hydrocarbon oils of animal origin, such as perhydrosqualene;
- hydrocarbon oils of plant origin, such as liquid triglycerides of fatty acids containing from 4 to 10 carbon atoms, for instance heptanoic or octanoic acid triglycerides, or alternatively, for example, sunflower oil, corn oil, soybean oil, marrow oil, grapeseed oil, sesame seed oil, hazelnut oil, apricot oil, macadamia oil, arara oil, castor oil, avocado oil, caprylic/capric acid triglycerides, for instance those sold by Stearineries Dubois or those sold under the names Miglyol 810, 812 and 818 by Dynamit Nobel, jojoba oil and shea butter oil;
- synthetic esters and ethers, especially of fatty acids, for instance the oils of formulae R1 COOR2 and R1OR2 in which R1 represents a fatty acid residue containing from 8 to 29 carbon atoms and R2 represents a branched or unbranched hydrocarbon-based chain containing from 3 to 30 carbon atoms, for instance purcellin oil, isononyl isononanoate, isopropyl myristate, 2-ethylhexyl palmitate, 2-octyldodecyl stearate, 2-octyldodecyl erucate or isostearyl isostearate; hydroxylated esters, for instance isostearyl lactate, octyl hydroxystearate, octyldodecyl hydroxystearate, diisostearyl malate or triisocetyl citrate; fatty alcohol heptanoates, octanoates or decanoates; polyol esters, for instance propylene glycol dioctanoate, neopentyl glycol diheptanoate and diethylene glycol diisononanoate; and pentaerythritol esters, for instance pentaerythrityl tetraisostearate;
- straight or branched hydrocarbons of inorganic or synthetic origin, such as volatile or non-volatile liquid paraffins, and derivatives thereof, petroleum jelly, polydecenes, and hydrogenated polyisobutene such as Parleam oil;
- fatty alcohols having from 8 to 26 carbon atoms, such as cetyl alcohol, stearyl alcohol and their mixture (cetearyl alcohol), octyldodecanol, 2-butyloctanol, 2-hexyldecanol, 2-undecylpentadecanol, oleyl alcohol or linoleyl alcohol;
- partially hydrocarbon-based and/or silicone-based fluoro oils, for instance those described in document JP-A-2-295 912;
- silicone oils, for instance volatile or non-volatile polymethylsiloxanes (PDMSs) with a straight or cyclic silicone chain, which are liquid or pasty at room temperature, especially cyclopolydimethylsiloxanes (cyclomethicones) such as cyclohexasiloxane; polydimethylsiloxanes containing alkyl, alkoxy or phenyl groups, which are pendent or at the end of a silicone chain, these groups containing from 2 to 24 carbon atoms; phenyl silicones, for instance phenyl trimethicones, phenyl dimethicones, phenyltrimethylsiloxydiphenylsiloxanes, diphenyl dimethicones, diphenylmethyldiphenyltrisiloxanes or 2-phenylethyl trimethylsiloxy silicates, and polymethylphenylsiloxanes; and
- mixtures thereof.

In the list of the above-mentioned oils, the term "hydrocarbon oil" is understood to mean any oil predominantly containing carbon and hydrogen atoms, and optionally ester, ether, fluoro, carboxylic acid and/or alcohol groups.

The composition of the invention comprises an aqueous phase.

Preferably, said aqueous phase comprised at least 70%, more preferably at least 80%, even more preferably at least 90%, advantageously at least 95%, for example at least 99%, of water by weight relative to the total weight of the aqueous phase.

Preferably, the composition of the invention comprises from 30% to 60% of water by weight relative to the total weight of the composition.

According to one embodiment, the composition of the invention is a water-in-oil (W/O) or oil-in-water (O/W) emulsion, preferably an O/W emulsion.

The proportion of the oil phase of the emulsion may range from 2% to 80% by weight and preferably from 5% to 50% by weight relative to the total weight of the composition.

The emulsions generally contain at least one emulsifier chosen from amphoteric, anionic, cationic or non-ionic emulsifiers, used alone or as a mixture, and optionally a co-emulsifier. The emulsifiers are appropriately chosen according to the emulsion to be obtained (W/O or O/W). The emulsifier and the co-emulsifier are generally present in the composition in a proportion ranging from 0.3% to 30% by weight and preferably from 0.5% to 20% by weight relative to the total weight of the composition.

For W/O emulsions, examples of emulsifiers that may be mentioned include dimethicone copolyols, such as the mixture of cyclomethicone and dimethicone copolyol sold under the name DC 5225 C by Dow Corning, and alkyl dimethicone copolyols such as the lauryl methicone copolyol sold under the name Dow Corning 5200 Formulation Aid by Dow Corning, and the cetyl dimethicone copolyol sold under the name Abil EM 90® by Goldschmidt. A crosslinked elastomeric solid organopolysiloxane containing at least one oxyalkylenated group, such as those obtained according to the procedure of Examples 3, 4 and 8 of document US-A-5 412 004 and of the examples of document US-A-5 811 487, especially the product of Example 3 (synthesis example) of patent US-A-5 412 004, such as the product sold under the reference KSG 21 by Shin-Etsu, may also be used as surfactant for W/O emulsions.

For O/W emulsions, examples of emulsifiers that may be mentioned include non-ionic emulsifiers such as oxyalkylenated (more particularly polyoxyethylenated) fatty acid esters of glycerol; oxyalkylenated fatty acid esters of sorbitan; oxyalkylenated (oxyethylenated and/or oxypropylenated) fatty acid esters; oxyalkylenated (oxyethylenated and/or oxypropylenated) fatty alcohol ethers; sugar esters such as sucrose stearate; and mixtures thereof, such as the mixture of glyceryl stearate and PEG-40 stearate.

According to a preferred embodiment, the composition of the invention comprises:
- from 4% to 25%, preferably from 5% to 20%, more preferably from 5% to 15%, of fatty acid,
- from 0.1% to 10%, preferably from 0.1% to 8%, advantageously from 0.1% to 5%, specifically from 0.1% to 3%, of basic agent,
- from 1% to 25%, preferably from 5% to 15%, advantageously from 7.5% to 12.5%, of UV absorption agent,
- from 0.01% to 30%, preferably from 0.01% to 20%, better still from 0.05% to 10%, more preferably from 0.1% to 5% and for example from 0.2% to 1%, of hydrophobic silica aerogel particles, and
- from 40% to 50% of water,
by weight relative to the total weight of the composition.

In the above paragraph, the term "fatty acid" is understood to mean the total amount of fatty acid, in the form of a soap as well as in the free form.

In the above paragraph, the term "UV absorption agent" is understood to mean the mixture of UV absorption agents present in the composition (i.e. the lipophilic UV absorption agent and the other optional UV absorption agents).

The present invention also relates to a cosmetic composition comprising, in a physiologically acceptable medium, a composition according to the invention.

The cosmetic composition of the invention may be also called a cosmetic sunscreen composition.

The composition of the invention is generally suitable for topical application to the skin and thus generally comprises a physiologically acceptable medium, i.e. a medium that is compatible with the skin and/or its integuments. It is preferably a cosmetically acceptable medium, i.e. a medium which has a pleasant colour, odour and feel and which does not cause any unacceptable discomfort (stinging, tautness or redness) liable to discourage the consumer from using this composition.

The cosmetic composition of the present invention may be any type of cosmetic composition such as a foundation, a face powder, an eye shadow, a concealer product, a blusher, a lipstick, a lip balm, a lip gloss, a lip pencil, an eye pencil, an eyeliner, a mascara, a body makeup product, a skin colouring product, a care product such as a care cream, a tinted cream or an antisun product, preferably a sunscreen care product such as a sunscreen care cream.

The present application also discloses a non-therapeutic cosmetic method for caring and/or making up the skin, comprising the topical application to said skin of a cosmetic composition according to the invention.

The present application also discloses a non-therapeutic cosmetic method for making the skin matt and/or for reducing its shine, comprising the topical application to said skin of a cosmetic composition according to the invention.

### Preparation method

The present application also discloses the preparation method of the composition of the invention, comprising:
- the preparation of the aqueous phase,
- the slow addition of the lipophilic ingredients to the aqueous phase under homogenization,
- the addition of hydrophobic silica aerogel particles in the form of a finely dispersed mixture comprising water and an organic solvent, and eventually
- the addition of additional ingredients.

The aqueous phase comprises water and the eventual hydrophilic additional ingredients. The aqueous phase is generally heated to a temperature comprised from 60°C to 100°C prior to the addition of the lipophilic ingredients.

The lipophilic ingredients, forming an oil phase, comprise the lipophilic UV absorption agent and the eventual other lipophilic additional ingredients. The lipophilic ingredients are typically previously mixed together and melted at a temperature comprised from 70°C to 85°C, prior to the addition of the resulting oil phase to the aqueous phase.

After the addition of the lipophilic ingredients to the aqueous phase, the mixture obtained is generally slowly allowed to cool to room temperature.

The hydrophobic silica aerogel particles are generally added at around 25°C to 30°C. Once the hydrophobic silica aerogel particles are added, the mixture is generally mixed at moderate speed.

The additional ingredients that may be added in the last step of the preparation method may be the filler other than the hydrophobic silica aerogel particles, color, fragrance, and any other additional ingredients describe above.

### Examples

- Stearic acid: STEARINE TP 1200 PASTILLES by STEARINERIE DUBOIS
- Triethanol amine:
- Lipophilic Sunscreens:
   - Butyl Methoxy Dibenzoylmethane (avobenzone): Parsol 1789 by DSM NUTRITIONAL PRODUCTS
   - 2-ethylhexyl α-cyano-β,β'-diphenylacrylate (octocrylene): UVINUL N 539® by BASF
   - drometrizole trisiloxane: MEXORYL XL® by CHIMEX
- Hydrophilic Sunscreen:
   - phenyl benzimidazole sulphonic acid: EUSOLEX 232® by MERCK
- Niacinamide: Vitamin B3 by WESTERN DRUGS
- TiO₂: Hombitan FF Pharma by SACHTLEBEN
- Silica silylate: Aerogel VM-2270 by DOW CORNING
- Silica: Sunsphere/SOLESPHERE H 51 by ASAHI GLASS

The following formulations were prepared following the standard process of soap based emulsions. The water phase was prepared at 80°C, followed by slow addition of the oil phase which was separately melted at 75-80°C along with the sunscreens, under homogenization. Next, it was slowly allowed to cool to room temperature. At around 25-30°C, aerogel phase in the form of a finely dispersed mixture in water was introduced and further mixed under moderate speed.

According to this general procedure, partially neutralized stearic acid and triethanol amine stearate soap-based sunscreen compositions in the form of oil-in-water (O/W) emulsions having the components and the quantities specified below were prepared.

### Example 1

| INCI Name (EU) | w/w % |
|---|---|
| | |
| Stearic acid (about 53% stearic acid, 44% palmitic acid, 3% myristic acid, by weight) | 9.0 |
| Triethanolamine | 0.50 |
| Butyl Methoxy Dibenzoylmethane | 3 |
| Niacinamide | 2.0 |
| TiO₂ | 0.25 |
| Silica silylate | 0.25 |
| Silica | 2 |
| Water | Q.S to 100 |

### Example 2

| INCI Name (EU) | w/w % |
|---|---|
| | |
| Stearic acid | 6.0 |
| Triethanolamine | 0.35 |
| Butyl Methoxy Dibenzoylmethane 2-ethylhexyl α-cyano-β,β'-diphenylacrylate Drometrizole trisiloxane | 3 4.5 1 |
| Phenyl benzimidazole sulphonic acid | 1 |
| Niacinamide | 2.0 |
| TiO₂ | 0.25 |
| Silica silylate | 0.25 |
| Silica | 2 |
| Water | Q.S to 100 |

### Example 3

| INCI Name (EU) | w/w % |
|---|---|
| | |
| Stearic acid | 9 |
| Triethanolamine | 0.35 |
| Butyl Methoxy Dibenzoylmethane 2-ethylhexyl α-cyano-β,β'-diphenylacrylate Drometrizole trisiloxane | 3 4.5 1 |
| Phenyl benzimidazole sulphonic acid | 1 |
| Niacinamide | 2.0 |
| TiO₂ | 0.25 |
| Silica silylate | 1 |
| Silica | 2 |
| Water | Q.S to 100 |

When it is applied to the skin, the compositions of examples 1, 2 and 3 according to the invention provide lower shine of the skin with respect to the same composition without silica silylate.

When it is applied to the skin, the compositions of examples 1, 2 and 3 according to the invention also reduce the production of sebum of the skin with respect to the same composition without silica silylate.

Thus, when it is applied to the skin, the composition according to the invention makes it possible to improve skin mattness, to reduce the skin shine, and to reduce the production of sebum of the skin, with respect to the composition of prior art.

## Claims

1. Composition comprising:
a) one or more fatty acids selected from the group consisting of straight or branched saturated fatty acids in C₈-C₃₀,
b) at least one basic agent,
c) at least one lipophilic UV absorption agent,
d) at least hydrophobic silica aerogel particles, and
e) at least one aqueous phase,
wherein said fatty acid(s) is(are) preset in the composition in an amount comprised from 4% to 25% by weight relative to the total weight of the composition.

2. Composition according to claim 1, wherein the fatty acid is in C₁₂-C₂₂, preferably in C₁₂-C₂₀, advantageously in C₁₄-C₁₈.

3. Composition according to claim 1 or 2, wherein the fatty acid is stearic acid.

4. Composition according to any one of claims 1 to 3, wherein the basic agent is selected from the group consisting of alkali metal hydroxides, alkaline earth metal hydroxides, ammonium hydroxide, organic bases, and mixtures thereof.

5. Composition according to claim 4, wherein the basic agent is an amine or an alkanolamine, such as triethanolamine.

6. Composition according to any one of claims 1 to 5, wherein the fatty acid is stearic acid and the basic agent is triethanolamine.

7. Composition according to any one of claims 1 to 6, wherein the lipophilic UV absorption agent is selected from the group consisting of dibenzoylmethane derivatives, β,β-diphenylacrylate derivatives, phenyl benzotriazole derivatives, and mixtures thereof.

8. Composition according to any one of claims 1 to 7, further comprising at least one hydrophilic UV absorption agent.

9. Composition according to any one of claims 1 to 8, wherein the hydrophobic silica aerogel particles are silylated silica aerogel particles.

10. Composition according to any one of claims 1 to 9, further comprising at least one skin lightening agent.

11. Composition according to any one of claims 1 to 10, further comprising at least one filler other than d).

12. Composition according to claim 11, wherein the filler other than d) is selected from the group consisting of silica other than hydrophobic silica aerogel particles, starch, talc, perlite, and mixtures thereof.

13. Composition according to any one of claims 1 to 12, comprising:
- from 4% to 25% of fatty acid,
- from 0.1 % to 10% of basic agent,
- from 1% to 25% of UV absorption agent,
- from 0.1% to 20% of hydrophobic silica aerogel particles, and
- from 40% to 50% of water,
by weight relative to the total weight of the composition.

## Patentansprüche

1. Zusammensetzung, die umfasst:
a) eine oder mehrere Fettsäuren, die aus der Gruppe ausgewählt sind, bestehend aus geraden oder verzweigten gesättigten Fettsäuren in C₈-C₃₀,
b) zumindest ein basisches Mittel,
c) zumindest ein lipophiles UV-Absorptionsmittel,
d) zumindest hydrophobe Siliciumdioxid-Aerogelpartikel, und
e) zumindest eine wässrige Phase,
wobei die eine oder mehreren Fettsäuren in der Zusammensetzung in einer Menge von 4 Gew.-% bis 25 Gew.-% bezogen auf das Gesamtgewicht der Zusammensetzung vorhanden sind.

2. Zusammensetzung nach Anspruch 1, wobei die Fettsäure in C₁₂-C₂₂, vorzugsweise in C₁₂-C₂₀, vorteilhafterweise in C₁₄-C₁₈, liegt.

3. Zusammensetzung nach Anspruch 1 oder 2, wobei die Fettsäure Stearinsäure ist.

4. Zusammensetzung nach einem der Ansprüche 1 bis 3, wobei das basische Mittel aus der Gruppe ausgewählt ist, bestehend aus Alkalimetallhydroxiden, Erdalkalimetallhydroxiden, Ammoniumhydroxid, organischen Basen und Mischungen davon.

5. Zusammensetzung nach Anspruch 4, wobei das basische Mittel ein Amin oder ein Alkanolamin wie z. B. Triethanolamin ist.

6. Zusammensetzung nach einem der Ansprüche 1 bis 5, wobei die Fettsäure Stearinsäure ist und das basische Mittel Triethanolamin ist.

7. Zusammensetzung nach einem der Ansprüche 1 bis 6, wobei das lipophile UV-Absorptionsmittel aus der Gruppe ausgewählt ist, bestehend aus Dibenzoylmethanderivaten, β,β-Diphenylacrylatderivaten, Phenylbenzotriazolderivaten und Mischungen davon.

8. Zusammensetzung nach einem der Ansprüche 1 bis 7, die ferner zumindest ein hydrophiles UV-Absorptionsmittel umfasst.

9. Zusammensetzung nach einem der Ansprüche 1 bis 8, wobei die hydrophoben Siliciumdioxid-Aerogelpartikel silylierte Siliciumdioxid-Aerogelpartikel sind.

10. Zusammensetzung nach einem der Ansprüche 1 bis 9, die ferner zumindest ein Hautaufhellungsmittel umfasst.

11. Zusammensetzung nach einem der Ansprüche 1 bis 10, die ferner zumindest einen anderen Füllstoff als d) umfasst.

12. Zusammensetzung nach Anspruch 11, wobei der andere Füllstoff als d) aus der Gruppe ausgewählt ist, bestehend aus anderem Siliciumdioxid als hydrophoben Siliciumdioxid-Aerogelpartikeln, Stärke, Talk, Perlit und Mischungen davon.

13. Zusammensetzung nach einem der Ansprüche 1 bis 12, die umfasst:
- 4 Gew.-% bis 25 Gew.-% Fettsäure,
- 0,1 Gew.-% bis 10 Gew.-% basisches Mittel,
- 1 Gew.-% bis 25 Gew.-% UV-Absorptionsmittel,
- 0,1 Gew.-% bis 20 Gew.-% hydrophobe Siliciumdioxid-Aerogelpartikel, und
- 40 Gew.-% bis 50 Gew.-% Wasser,
bezogen auf das Gesamtgewicht der Zusammensetzung.

## Revendications

1. Composition comprenant :
a) un ou plusieurs acides gras choisis dans le groupe constitué par les acides gras saturés linéaires ou ramifiés en C₈ à C₃₀,
b) au moins un agent basique,
c) au moins un agent lipophile absorbant les UV,
d) au moins des particules d'aérogel de silice hydrophobe, et
e) au moins une phase aqueuse,
dans laquelle le ou les acides gras sont prédéfinis dans la composition en une quantité comprise entre 4 % et 25 % en poids par rapport au poids total de la composition.

2. Composition selon la revendication 1, dans laquelle l'acide gras est en C₁₂ à C₂₂, de préférence en C₁₂ à C₂₀, avantageusement en C₁₄ à C₁₈.

3. Composition selon la revendication 1 ou 2, dans laquelle l'acide gras est l'acide stéarique.

4. Composition selon l'une quelconque des revendications 1 à 3, dans laquelle l'agent basique est choisi dans le groupe constitué par les hydroxydes de métal alcalin, les hydroxydes de métal alcalino-terreux, l'hydroxyde d'ammonium, les bases organiques, et leurs mélanges.

5. Composition selon la revendication 4, dans laquelle l'agent basique est une amine ou une alcanolamine, telle que la triéthanolamine.

6. Composition selon l'une quelconque des revendications 1 à 5, dans laquelle l'acide gras est l'acide stéarique et l'agent basique est la triéthanolamine.

7. Composition selon l'une quelconque des revendications 1 à 6, dans laquelle l'agent lipophile absorbant les UV est choisi dans le groupe constitué par les dérivés de dibenzoylméthane, les dérivés de β,β-diphénylacrylate, les dérivés de phénylbenzotriazole, et leurs mélanges.

8. Composition selon l'une quelconque des revendications 1 à 7, comprenant en outre au moins un agent hydrophile absorbant les UV.

9. Composition selon l'une quelconque des revendications 1 à 8, dans laquelle les particules d'aérogel de silice hydrophobe sont des particules d'aérogel de silice silylée.

10. Composition selon l'une quelconque des revendications 1 à 9, comprenant en outre au moins un agent éclaircissant la peau.

11. Composition selon l'une quelconque des revendications 1 à 10, comprenant en outre au moins une charge autre que d).

12. Composition selon la revendication 11, dans laquelle la charge autre que d) est choisie dans le groupe constitué par une silice autre que les particules d'aérogel de silice hydrophobe, l'amidon, le talc, la perlite, et leurs mélanges.

13. Composition selon l'une quelconque des revendications 1 à 12, comprenant :
- de 4 % à 25 % d'acide gras,
- de 0,1 % à 10 % d'agent basique,
- de 1 % à 25 % d'agent absorbant les UV,
- de 0,1 % à 20 % de particules d'aérogel de silice hydrophobe, et
- de 40 % à 50 % d'eau,
en poids par rapport au poids total de la composition.
